# EUROPEAN PATENT SPECIFICATION

(11) **EP 2 699 297 B1**
(45) Date of publication and mention of the grant of the patent: **08.06.2016**
(21) Application number: 12719078.3
(22) Date of filing: 11.04.2012
(51) Int. Cl.: A61M 16/06

(54) **ADJUSTMENT MECHANISM FOR HEADGEAR FOR USE IN PATIENT INTERFACE SYSTEM**
KOPFHALTERUNG MIT EINSTELLMECHANISMUS FÜR EIN PATIENTENSCHNITTSTELLENSYSTEM
MÉCANISME DE RÉGLAGE POUR UN SYSTÈME D'INTERFACE DE PATIENT

(30) Priority: 18.04.2011 US 201161476449 P
(43) Date of publication of application: 26.02.2014
(73) Proprietor: Koninklijke Philips N.V., 5656 AE Eindhoven (NL)
(72) Inventor: MATULA, Jerome, NL-5656 AE Eindhoven (NL); ANDREWS, Derrick, Blake, NL-5656 AE Eindhoven (NL); STARTARE, Anthony, Vincent, NL-5656 AE Eindhoven (NL)
(74) Representative: Steffen, Thomas
(86) International application number: PCT/IB2012/051763
(87) International publication number: WO 2012/143822

(56) References cited:
- WO-A1-2009/108994
- DE-U1-202008 012 163
- US-A1- 2010 031 963

## Description

### BACKGROUND OF THE INVENTION

### 1. Field of the Invention

The present invention relates to patient interface systems for communicating a flow of gas with an airway of a user, and, in particular, to an adjustment mechanism for use with a headgear in securing a patient interface device of a patient interface system to a head of a user.

### 2. Description of the Related Art

There are numerous situations where it is necessary or desirable to deliver a flow of breathing gas non-invasively to the airway of a patient, i.e., without intubating the patient or surgically inserting a tracheal tube in their esophagus. For example, it is known to ventilate a patient using a technique known as non-invasive ventilation. It is also known to deliver continuous positive airway pressure (CPAP) or variable airway pressure, which varies with the patient's respiratory cycle, to treat a medical disorder, such as sleep apnea syndrome, in particular, obstructive sleep apnea (OSA), or congestive heart failure.

Non-invasive ventilation and pressure support therapies involve the placement of a patient interface device including a mask component on the face of a patient. The mask component may be, without limitation, a nasal mask that covers the patient's nose, a nasal cushion having nasal prongs that are received within the patient's nares, a nasal/oral mask that covers the nose and mouth, or a full face mask that covers the patient's face. The patient interface device interfaces the ventilator or pressure support device with the airway of the patient, so that a flow of breathing gas can be delivered from the pressure/flow generating device to the airway of the patient. It is known to maintain such devices on the face of a wearer by a headgear having one or more straps adapted to fit over/around the patient's head.

Because such patient interface devices are typically worn for an extended period of time, it is important for the headgear to maintain the mask component of the device in a tight enough seal against the patient's face without discomfort. In order to maintain such desired fitment, one or more straps of the headgear must typically be carefully adjusted to desired tensions. However, removal of the mask component from the patient commonly requires such strap(s) to be loosened or disengaged from the mask. Presently, the most common device for accomplishing this is a quick-release clip which disengages from the mask. The quick-release method generally requires the patient to align two mating components while the mask is on the patient's head. Such action is not intuitive and can be particularly difficult when the patient is sleepy and in the dark. Such connection also adds complexity to the mask and has the ability to be incorrectly or incompletely engaged which can result in poor sealing of the mask to the patient.

An alternative to the quick-release method is to have no clip and instead disconnect the headgear from the mask by undoing Velcro® straps. Such method requires the patient to reattach and readjust the straps each time the mask is placed on the patient. Such adjustments are time consuming and can result in the patient adjusting the straps incorrectly, either by over or under tightening.

US 2010/0031963 A1 discloses a respiratory mask that includes a single-piece first cushion ring defining an airflow channel and having a front cover-engaging end portion an a rear abutment end portion adapted to abut against a user's face below the forehead, a cover shell having a cover plate engaged to the front cover-engaging end portion and provided with a connecting hole that is adapted to be connected to an air supply source, a resilient forehead support connected to a top end of the front cover-engaging end portion and bendable toward the rear abutment end portion, a resilient forehead abutment body connected to a top end of and supported by the resilient forehead support and including a second cushion ring adapted to abut against the forehead of the user, and an adjustable head strap unit connected to the first cover shell and the resilient forehead abutment body.

DE 20 2008 012 0163 U1 discloses a quick release and locking mechanism for a strap of a helmet.

### SUMMARY OF THE INVENTION

Accordingly, it is an object of one or more embodiments of the present invention to overcome the shortcomings of existing devices by providing a patient interface device that includes a patient sealing assembly, such as, without limitation, a mask having a rigid shell, and a sealing element, such as a cushion, coupled to the rigid shell. It is particularly an object of the present invention to provide a headgear for a patient interface with a quick release and locking mechanism which does not require to disconnect any portion of the headgear assembly from the mask.

This object is solved by a headgear assembly according to claim 1. The headgear assembly includes an adjustment mechanism that allows for a user to loosen a strap via a folding mechanism which does not require removal of the headgear from the mask, either by manual methods or quick release clip. Instead, the adjustment mechanism allows the patient to remove the mask by unfolding the mechanism and making the associated strap longer. When the
patient places the mask back on, the associated strap can then be shortened to its original length by refolding the mechanism.

In an exemplary embodiment, the adjustment mechanism includes a first portion, a second portion, and a fastening mechanism. The first portion has a first end and an opposite second end , the first end coupled to the patient sealing assembly. The second portion has a first end and an opposite second end, the first end movably coupled to the second end of the first portion and the second end coupled to a portion of the headgear assembly. The second portion is movable among a first position in which the first and second portions are generally aligned and a second position in which the first and second portions are disposed generally parallel and the fastening mechanism is disposed to selectively fasten the first and second portions in the second position.

The headgear assembly may include a strap member and the first portion of the adjustment mechanism may include a number of rigid or semi-rigid elements coupled to, or disposed in or through, a first portion of the strap member. The second portion of the adjustment mechanism may include a number of rigid or semi-rigid elements coupled to, or disposed in or through, a second portion of the strap member. The first end of the second portion of the adjustment mechanism may be movably coupled to the second end of the first portion of the adjustment mechanism by a third portion of the strap member disposed between the first and second portions of the strap member.

The fastening mechanism may include a number of cooperating components disposed in a manner that selectively fastens the first and second portions of the adjustment mechanism in the second position. The number of cooperating components may include at least one of magnets, Velcro® strips, or snap mechanisms. The fastening mechanism may include a strap member coupled at or about one of the first end of the first portion and the second end of the second portion and selectively coupled at or about the other one of the first end of the first portion and the second end of the second portion.

The adjustment mechanism may include a third portion having a first end and an opposite second end, the first end movably coupled to the second end of the second portion of the adjustment mechanism.

These and other objects, features, and characteristics of the present invention, as well as the methods of operation and functions of the related elements of structure and the combination of parts and economies of manufacture, will become more apparent upon consideration of the following description and the appended claims with reference to the accompanying drawings, all of which form a part of this specification, wherein like reference numerals designate corresponding parts in the various figures. It is to be expressly understood, however, that the drawings are for the purpose of illustration and description only and are not intended as a definition of the limits of the invention.

### BRIEF DESCRIPTION OF THE DRAWINGS

FIGS. 1 and 2 are front and side schematic diagrams, respectively, of a system adapted to provide a regimen of respiratory therapy to a patient according to one embodiment of the invention shown disposed on the head of a patient;
FIG. 3 is an isometric view of a strap adjustment mechanism according to one embodiment of the invention shown in an unfastened, fully extended position;
FIG. 4 is an isometric view of the strap adjustment mechanism of FIG. 3 shown in a unfastened, partially extended position;
FIG. 5 is an isometric view of the strap adjustment mechanism of FIG. 3 shown in a fastened, unextended position;
FIG. 6 is an isometric views of a strap adjustment mechanism according to another embodiment of the invention shown in an unfastened, fully extended position;
FIG. 7 is an isometric view of the strap adjustment mechanism of FIG. 6 shown in a unfastened, partially extended position; and
FIG. 8 is an isometric view of the strap adjustment mechanism of FIG. 6 shown in a fastened, unextended position.

### DETAILED DESCRIPTION OF THE EXEMPLARY EMBODIMENTS

Directional phrases used herein, such as, for example and without limitation, top, bottom, left, right, upper, lower, front, back, and derivatives thereof, relate to the orientation of the elements shown in the drawings and are not limiting upon the claims unless expressly recited therein. As used herein, the singular form of "a", "an", and "the" include plural references unless the context clearly dictates otherwise. As used herein, the statement that two or more parts or components are "coupled" shall mean that the parts are joined or operate together either directly or indirectly, i.e., through one or more intermediate parts or components, so long as a link occurs. As used herein, "directly coupled" means that two elements are directly in contact with each other. As used herein, "fixedly coupled" or "fixed" means that two components are coupled so as to move as one while maintaining a constant orientation relative to each other.

As employed herein, the word "unitary" means a component is created as a single piece or unit. That is, a component that includes pieces that are created separately and then coupled together as a unit is not a "unitary" component or body. As employed herein, the statement that two or more parts or components "engage" one another shall mean that the parts exert a force against one another either directly or through one or more intermediate parts or components. As employed herein, the term "number" shall mean one or an integer greater than one (i.e., a plurality).

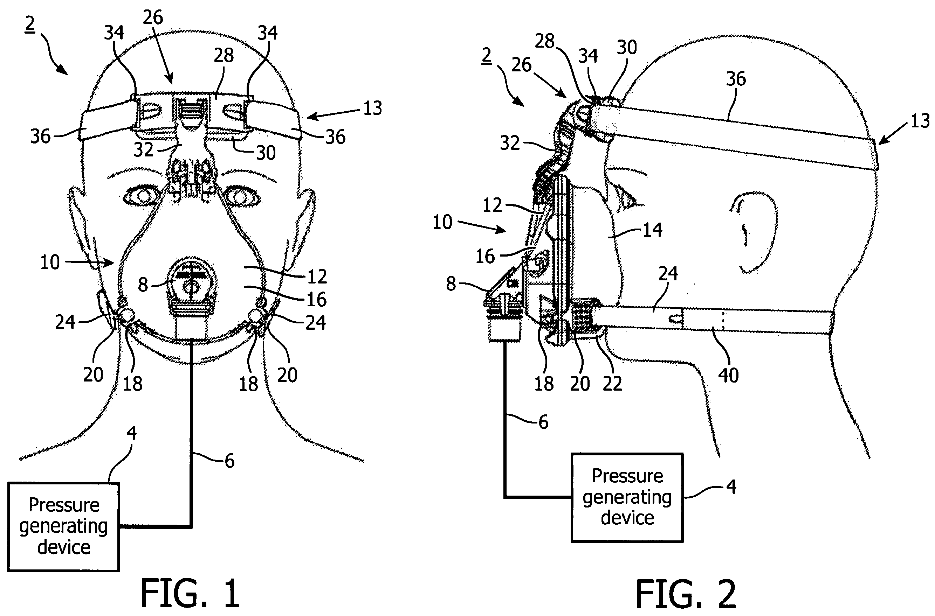

An exemplary embodiment of a system 2 adapted to provide a regimen of respiratory therapy to a patient according to the principles of the present invention is generally shown disposed on a head of a patient in FIGS. 1 and 2. System 2 includes a pressure generating device 4, a delivery conduit 6 coupled to an elbow connector 8, and a patient interface device 10. Pressure generating device 4 is structured to generate a flow of breathing gas and may include, without limitation, ventilators, constant pressure support devices (such as a continuous positive airway pressure device, or CPAP device), variable pressure devices (e.g., BiPAP®, Bi-Flex®, or C-Flex™ devices manufactured and distributed by Philips Respironics of Murrysville, Pennsylvania), and auto-titration pressure support devices. Delivery conduit 6 is structured to communicate the flow of breathing gas from pressure generating device 4 to patient interface device 10 through elbow connector 8 and may include, without limitation, flexible tubing. Elbow connector 8 is preferably coupled to patient interface device 10. Delivery conduit 6, elbow connector 8 and patient interface device 10 are often collectively referred to as a patient circuit (not numbered).

Patient interface device 10 includes a patient sealing assembly, which in the illustrated embodiment is a mask 12, and a headgear assembly 13 that secures the patient sealing assembly to the head of a patient. In the illustrated exemplary embodiment of FIGS. 1 and 2, mask 12 is in the form of a nasal/oral mask that seals over the nose and mouth. However, any type of patient sealing assembly, such as a nasal mask that seals over the nose only, which facilitates the delivery of the flow of breathing gas to the airway of a patient, may be substituted for mask 12 while remaining within the scope of the present invention. Also, although shown as a four-point headgear in FIGS. 1 and 2, any type of headgear that utilizes one or more straps to secure a patient sealing assembly to the head of a patient, such as a two-point headgear, may be substituted for headgear assembly 13 while remaining within the scope of the present invention.

Mask 12 includes a sealing means for creating a seal against the skin. In the illustrated embodiment, the sealing means is a cushion 14 coupled to a rigid shell 16. The present invention contemplates that other techniques for creating a seal against the user may be employed, such as nasal pillows or cannula that seal over the nares, multi-flap cushions, and cushions having any number of configurations and materials.

An opening (not numbered) in shell 16 is provided to which elbow connector 8 is coupled. The opening allows the flow of breathing gas from pressure generating device 4 to be communicated to an interior space (not numbered) defined by shell 16 and cushion 14, and then to the airway of a patient on which patient interface device 10 is disposed. In addition, cushion 14 includes a sealing surface (not numbered) that is structured to engage the face of a patient when patient interface device 10 is donned by the patient, such as shown, for example, in FIGS 1 and 2.

Shell 16 includes first and second slots 18 each structured to receive and hold a catch of clip element 20. In the exemplary embodiment, slots 18 and clip elements 20 are structured in the form of a ball and socket configuration. Each clip element 20 also includes a loop 22 for receiving a respective lower headgear strap 24 of headgear assembly 13 used to secure patient interface device 10 to the head of the patient.

Patient interface device 10 further includes a forehead support 26 that, in the illustrated embodiment, includes a support frame 28 having a forehead cushion 30 coupled thereto. Forehead support 26 is structured to provide additional support for patient interface device 10 by engaging the forehead of the patient. Support frame 28 is coupled to an upper arm 32, which in turn is coupled to shell 16. In the illustrated exemplary embodiment of FIGS. 1 and 2, support frame 28 includes loops 34 provided at opposite ends thereof. Each loop 34 is structured to receive a respective upper headgear strap 36 of headgear assembly 13. As is known, lower headgear straps 24 and upper headgear straps 36 enable the headgear component of which they are a part to secure the patient sealing assembly to the patient's head. It is to be understood that the present invention contemplates any technique or configuration for coupling the headgear to the support frame either through the use of intermediate coupling mechanisms or through direct coupling without the use of intermediate coupling mechanisms.

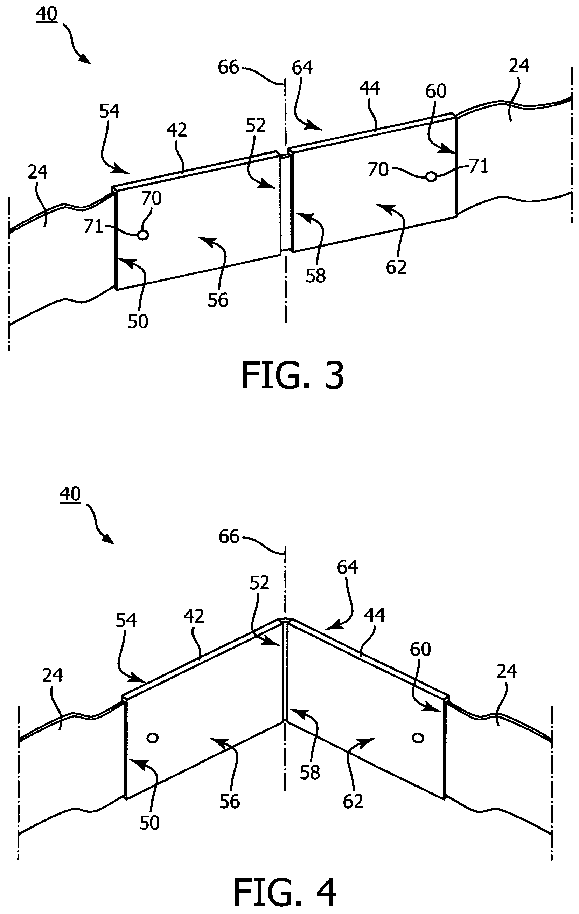

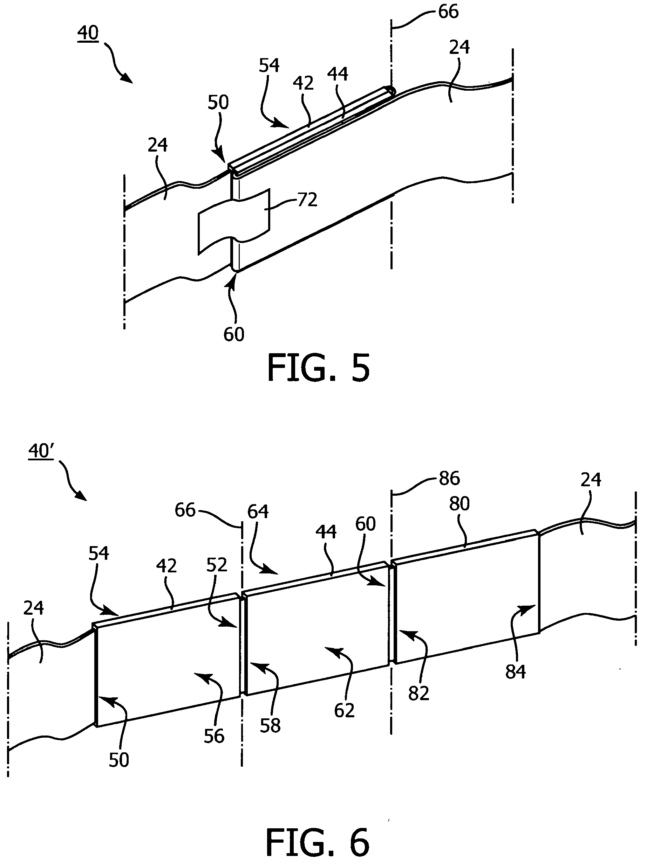

To facilitate installation or removal of patient interface device 10 onto or from the head of a patient, one or both of headgear straps 24, 36 may include one or more adjustment mechanisms, such as strap adjustment mechanism 40 in lower headgear strap 24 of FIGS. 1 and 2. FIGS. 3, 4 and 5 show an adjustment mechanism 40 according to an exemplary embodiment of the present invention in "unfastened" positions (FIGS. 3 and 4) and a "fastened" position (FIG. 5).

Referring to FIG. 3 which shows strap adjustment mechanism 40 in an "unfastened", fully extended position, adjustment mechanism 40 includes a first portion 42 and a second portion 44. First portion 42 is preferably generally planar in shape and includes a first end 50, an opposite second end 52, a patient facing side 54, and an opposite outer side 56. Continuing to refer to FIG. 3, second portion 44 is also likewise preferably generally planar in shape and includes a first end 58, an opposite second end 60, a patient facing side 62, and an opposite outer side 64.

First end 58 of second portion 44 is movably coupled (in the illustrated example embodiment pivotally coupled) to second end 52 of first portion 42 such that first and second portions 42 and 44 move about, and thus are generally hinged about an axis 66. Each of portions 42 and 44 are preferably generally semi-rigid or rigid and may be formed in a number of different manners in accordance with the present invention. For example, first and second portions 42 and 44 may be formed as stiffened or reinforced portions of a continuous strap member, such as, for example, lower headgear strap 24 shown in the illustrated example. Such stiffening of selected portions of a continuous strap may be accomplished in a variety of different manners, for example, without limitation, by: attaching rigid or semi-rigid stiffeners in or to the strap by sewing, molding, or other suitable means; coating or impregnating the strap with a stiffer material such as silicone or other suitable material; or adding stitching to the strap. Alternately, one or both of first and second portions may be formed separately from the strap and then placed in, and coupled to respective portions of the strap or placed over and coupled to selected portions of the strap. In embodiments where first and second portion 42 and 44 are formed as portions of a continuous strap or placed over portions of a continuous strap, a generally vertical line of unmodified strap material separating first and second portions 42 and 44 would preferably be left unstiffened to provide an area acting as a hinge between the portions. Alternately, where first and second portions 42 and 44 replace a section of a strap, first and second portions 42 and 44 are preferably directly coupled via a hinge mechanism generally formed as portions of one or both of first and second portions 42, 44.

Regardless of how formed, operation of adjustment mechanism 40 would be generally the same. As shown in FIGS 3-5, first end 50 of first portion 42 and second end 60 of second portion 44, respectively, are each coupled to a separate portion of lower headgear strap 24. The movement of second portion 44 with respect to first portion 42 about axis 66 provides for second portion 44 to be positioned in a first position in which adjustment mechanism 40 is "unfastened" and fully extended such that second portion 44 and first portion 42 are generally aligned, as shown in FIG. 3. In such first position, strap 24 is preferably lengthened a sufficient amount to allow for the strap to be slipped over the head of the patient, thus allowing patient interface device 10 to be removed without needing to disconnect any portion of headgear assembly 13 from mask 12.

Upon placing patient interface device 10 onto the head of the patient, second portion 44 may be moved from the first position, as shown in FIG. 3, to a second position, as shown in FIG. 5, in which adjustment mechanism 40 is contracted such that second portion 44 is disposed generally parallel to first portion 42 and generally "fastened" in such second position via one or more fastening mechanisms 70 (FIGS. 3 and 4). FIG. 4 shows adjustment mechanism 40 disposed in an intermediate position between the first and second positions of FIGS. 3 and 5. Shown schematically in FIGS. 3 and 4, fastening mechanism 70 may include a number of cooperating components 71 preferably positioned in a manner such that they act to keep first and second portions 42, 44 of adjustment mechanism 40 "fastened" in the second position of FIG. 5 until the patient desires to "unfasten" adjustment mechanism 40. Examples of suitable cooperating components 71 include, for example, without limitation, magnets, Velcro® strips, and snap mechanisms. It is t be appreciated that the positioning of cooperating components 71 shown in the FIGS. is for example purposes only and is not intended to be limiting upon the invention as the quantity and placement of such components may be varied for a particular application. Also, such components may be disposed on or in first and second portions 42 and 44.

In place of, or in addition to cooperating components 71 which are generally concealed when adjustment mechanism 40 is positioned in the "fastened" position of FIG. 5, adjustment mechanism 40 may include one or more external fastening mechanisms, such as strap member 72 shown in the illustrated embodiment of FIG. 5. Strap member 72 acts to selectively couple second end 60 of second portion 44 generally to first end 50 of first portion 42 either through a direct coupling or by coupling to strap 24 adjacent first end 50. Such coupling may be achieved by fixedly coupling one end of strap 72 and selectively coupling the other end of strap 72, via magnets, Velcro® strips, snap mechanisms, or other suitable coupling mechanisms, or alternately, selectively coupling both ends of strap 72 via such selective coupling mechanisms. The redundant use of both fastening mechanisms 70 and 72 generally allows for each of such fastening mechanisms to be generally easier to separate or "unfasten", thus requiring less effort by a patient.

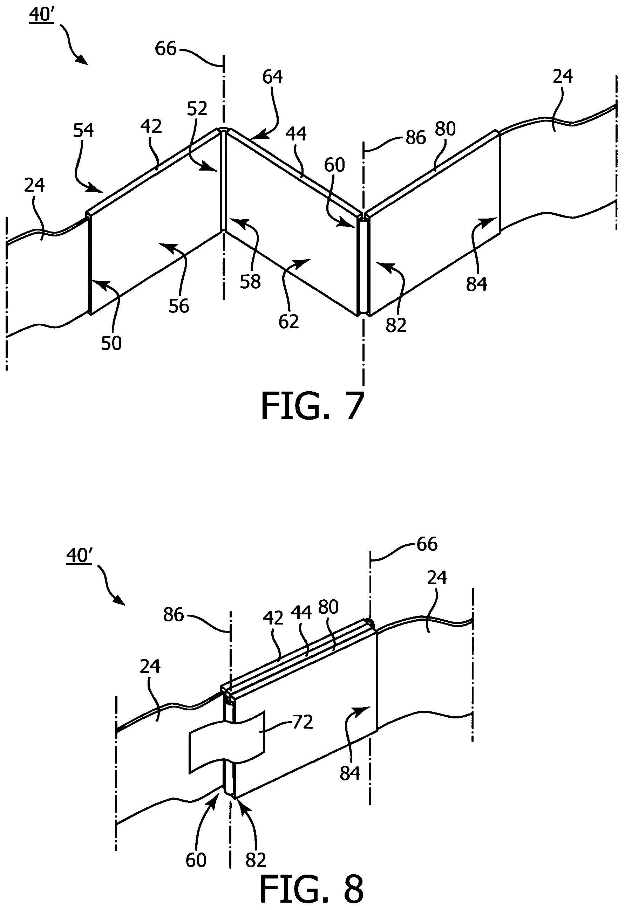

FIGS. 6-8 show an example of an adjustment mechanism 40' according to another exemplary embodiment of the present invention in a fully extended, partially extended, and contracted positions, respectively. Adjustment mechanism 40' includes all of the elements of adjustment mechanism 40 and further includes a third portion 80, preferably of similar construction as first and second portions 42 and 44, previously described. Third portion 80 includes a first end 82 movably coupled (in the illustrated embodiment pivotally coupled) to second end 60 of second portion 44 such that second and third portions 44 and 80 move about, and thus are generally hinged about a second axis 86. Third portion 80 further includes an opposite second end 84 coupled to a portion of strap 24. In use, adjustment mechanism 40' functions in a similar manner as adjustment mechanism 40 previously discussed, however third portion 80 helps to maintain proper alignment and prevent unwanted twisting of strap 24 which may occur with use of adjustment mechanism 40.

While an embodiment having an exemplary adjustment mechanism has been described in detail herein, it should be understood that other embodiments are also possible within the scope of the present invention. Although shown in as being used in conjunction with only one side of lower headgear strap 24, it is to be understood that the present invention contemplates that the strap adjustment mechanism may be used with either or both sides of a headgear assembly. Additionally, the present invention also contemplates that multiple adjustment mechanisms could be used in series on a single strap to provide for greater selectable elongation of a particular strap in a headgear assembly.

From the description herein, it is to be appreciated that strap adjustment mechanism 40 allows for a user to loosen a strap via a folding mechanism which does not require removal of the headgear from the mask, either by manual methods or quick release clip. Instead, adjustment mechanism 40 allows the patient to remove the mask by unfolding the mechanism and making the associated strap longer. When the patient places the mask back on, the associated strap can then be shortened to its original length by refolding the mechanism.

Although the invention has been described in detail for the purpose of illustration based on what is currently considered to be the most practical and preferred embodiments, it is to be understood that such detail is solely for that purpose and that the invention is not limited to the disclosed embodiments, but, on the contrary, is intended to cover modifications and equivalent arrangements that are within the scope of the appended claims.

## Claims

1. A headgear assembly (13) for use in a patient interface device (10) for coupling a patient sealing assembly to the head of a patient, the patient sealing assembly adapted to communicate a flow of breathing gas with an airway of the patient, the headgear assembly (13) comprising:
(a) a strap member (24); and
(b) an adjustment mechanism (40, 40') comprising a fastening mechanism (70),
**characterized in that** the adjustment mechanism (40, 40') further comprises:
(1) a first portion (42) having a first end (50) and an opposite second end (52), the first end (50) adapted to be coupled to the patient sealing assembly;
(2) a second portion (44) having a first end (58) and an opposite second end (60), the first end (58) movably coupled to the second end (52) of the first portion (42) and the second end (60) coupled to a portion of the strap member (24);
wherein the second portion (44) is movable among a first position in which the first and second portions (42, 44) are generally aligned and a second position in which the first and second portions (42, 44) are disposed generally parallel, and wherein the fastening mechanism (70) is disposed to selectively fasten the first and second portions (42, 44) in the second position;
wherein the strap member (24) is a continuous strap member (24), and wherein the first and second portions (42, 44) are formed as stiffened or reinforced portions of the continuous strap member (24) or placed over portions of the continuous strap member (24).

2. The headgear assembly according to claim 1, wherein the first portion (42) of the adjustment mechanism (40, 40') comprises a number of rigid or semi-rigid elements coupled to, or disposed in or through, a first portion of the strap member (24); wherein the second portion (44) of the adjustment mechanism (40, 40') comprises a number of rigid or semi-rigid elements coupled to, or disposed in or through, a second portion of the strap member (24), and wherein the first end (58) of the second portion (44) of the adjustment mechanism (40, 40') is movably coupled to the second end (50) of the first portion (42) of the adjustment mechanism (40, 40') by a third portion of the strap member (24) disposed between the first and second portions (42, 44) of the strap member (24).

3. The headgear assembly according to claim 1, wherein the fastening mechanism (70) comprises a number of cooperating components (71) disposed in a manner that selectively fastens the first and second portions (42, 44) of the adjustment mechanism (40, 40') in the second position.

4. The headgear assembly according to claim 3 wherein the number of cooperating components (71) include at least one of magnets or snap mechanisms.

5. The headgear assembly according to claim 1, wherein the fastening mechanism (70) comprises a strap member (72) coupled at or about one of the first end (50) of the first portion (42) and the second end (60) of the second portion (44) and selectively coupled at or about the other one of the first end (50) of the first portion (42) and the second end (60) of the second portion (44).

6. The headgear assembly according to claim 1, wherein the adjustment mechanism (40') comprises a third portion (80) having a first end (82) and an opposite second end (84), the first end (82) movably coupled to the second end (60) of the second portion (44) of the adjustment mechanism (40').

7. A patient interface device (10), comprising:
(a) a patient sealing assembly adapted to communicate a flow of breathing gas with an airway of a patient; and
(b) a headgear assembly (13) according to any of claims 1 to 6 coupled to the patient sealing assembly.

8. The patient interface device according to claim 7, wherein the patient sealing assembly comprises a mask (12) having a rigid shell (16) and a sealing element (14) coupled to the rigid shell (16), and wherein the first end (50) of the first portion (42) is coupled to the rigid shell (16).

## Patentansprüche

1. Kopfhalterungsbaugruppe (13) zur Verwendung in einer Patientenschnittstellenvorrichtung (10) zum Koppeln einer Patientenabdichtungsbaugruppe mit dem Kopf eines Patienten, wobei die Patientenabdichtungsbaugruppe dafür ausgelegt ist, eine Atemgasströmung an einen Atemweg des Patienten zu übermitteln, wobei die Kopfhalterungsbaugruppe (13) Folgendes umfasst:
(a) ein Gurtelement (24); und
(b) einen Einstellmechanismus (40, 40') umfassend einen
Befestigungsmechanismus (70),
**dadurch gekennzeichnet, dass** der Einstellmechanismus (40, 40') weiterhin Folgendes umfasst:
(1) einen ersten Abschnitt (42) mit einem ersten Ende (50) und einem gegenüberliegenden zweiten Ende (52), wobei das erste Ende (50) dafür ausgelegt ist, mit der Patientenabdichtungsbaugruppe gekoppelt zu werden;
(2) einen zweiten Abschnitt (44) mit einem ersten Ende (58) und einem gegenüberliegenden zweiten Ende (60), wobei das erste Ende (58) beweglich mit dem zweiten Ende (52) des ersten Abschnitts (42) gekoppelt ist und das zweite Ende (60) mit einem Abschnitt des Gurtelements (24) gekoppelt ist;
wobei der zweite Abschnitt (44) zwischen einer ersten Position, in der der erste und der zweite Abschnitt (42, 44) im Allgemeinen in einer Linie liegen, und einer zweiten Position, in der der erste und der zweite Abschnitt (42, 44) im Allgemeinen parallel zueinander liegen, beweglich ist, und wobei der Befestigungsmechanismus (70) angeordnet ist, um den ersten und den zweite Abschnitt (42, 44) selektiv in der zweiten Position zu befestigen;
wobei das Gurtelement (24) ein kontinuierliches Gurtelement (24) ist, und wobei der erste und der zweite Abschnitt (42, 44) als versteifte oder verstärkte Abschnitte des kontinuierlichen Gurtelements (24) geformt sind oder über Abschnitten des kontinuierlichen Gurtelements (24) platziert sind.

2. Kopfhalterungsbaugruppe nach Anspruch 1, wobei der erste Abschnitt (42) des Einstellmechanismus (40, 40') eine Anzahl von steifen oder halbsteifen Elementen umfasst, die mit einem ersten Abschnitt des Gurtelements (24) gekoppelt sind oder darin oder durch diesen hindurch angeordnet sind; wobei der zweite Abschnitt (44) des Einstellmechanismus (40, 40') eine Anzahl von steifen oder halbsteifen Elementen umfasst, die mit einem zweiten Abschnitt des Gurtelements (24) gekoppelt sind oder darin oder durch diesen hindurch angeordnet sind, und wobei das erste Ende (58) des zweiten Abschnitts (44) des Einstellmechanismus (40, 40') beweglich mit dem zweiten Ende (50) des ersten Abschnitts (42) des Einstellmechanismus (40, 40') durch einen dritten Abschnitt des Gurtelements (24) gekoppelt ist, der zwischen dem ersten und dem zweiten Abschnitt (42, 44) des Gurtelements (24) angeordnet ist.

3. Kopfhalterungsbaugruppe nach Anspruch 1, wobei der Befestigungsmechanismus (70) eine Anzahl von zusammenwirkenden Komponenten (71) umfasst, die auf eine Weise angeordnet sind, die den ersten und den zweiten Abschnitt (42, 44) des Einstellmechanismus (40, 40') selektiv in der zweiten Position befestigt.

4. Kopfhalterungsbaugruppe nach Anspruch 3, wobei die Anzahl der zusammenwirkenden Komponenten (71) mindestens entweder Magnete oder Einschnappmechanismen umfassen.

5. Kopfhalterungsbaugruppe nach Anspruch 1, wobei der Befestigungsmechanismus (70) ein Gurtelement (72) umfasst, das an oder um entweder das erste Ende (50) des ersten Abschnitts (42) oder das zweite Ende (60) des zweiten Abschnitts (44) gekoppelt ist und selektiv an oder um das andere Ende, also das erste Ende (50) des ersten Abschnitts (42) oder das zweite Ende (60) des zweiten Abschnitts (44), gekoppelt ist.

6. Kopfhalterungsbaugruppe nach Anspruch 1, wobei der Einstellmechanismus (40') einen dritten Abschnitt (80) mit einem ersten Ende (82) und einem gegenüberliegenden zweiten Ende (84) umfasst, wobei das erste Ende (82) beweglich mit dem zweiten Ende (60) des zweiten Abschnitts (44) des Einstellmechanismus (40') gekoppelt ist.

7. Patientenschnittstellenvorrichtung (10), die Folgendes umfasst:
(a) eine Patientenabdichtungsbaugruppe, die dafür ausgelegt ist, eine Atemgasströmung an einen Atemweg eines Patienten zu übermitteln; und
(b) eine Kopfhalterungsbaugruppe (13) nach einem der Ansprüche 1 bis 6, die mit der Patientenabdichtungsbaugruppe gekoppelt ist.

8. Patientenschnittstellenvorrichtung nach Anspruch 7, wobei die Patientenabdichtungsbaugruppe eine Maske (12) mit einer steifen Schale (16) und einem Abdichtungselement (14) umfasst, das mit der steifen Schale (16) gekoppelt ist, und wobei das erste Ende (50) des ersten Abschnitts (42) mit der steifen Schale (16) gekoppelt ist.

## Revendications

1. Ensemble cranio-cervical (13) pour l'utilisation dans un dispositif d'interface pour patient (10) pour accoupler un ensemble d'étanchéité pour patient à la tête d'un patient, l'ensemble d'étanchéité pour patient étant adapté pour faire communiquer un écoulement de gaz respiratoire avec des voies respiratoires du patient, l'ensemble cranio-cervical (13) comprenant :
(a) un organe à sangle (24) ; et
(b) un mécanisme d'ajustement (40, 40') comprenant un mécanisme de fixation (70),
**caractérisé en ce que** le mécanisme d'ajustement (40, 40') comprend en outre :
(1) une première portion (42) possédant une première extrémité (50) et une seconde extrémité opposée (52), la première extrémité (50) étant adaptée pour être accouplée à l'ensemble d'étanchéité pour patient ;
(2) une deuxième portion (44) possédant une première extrémité (58) et une seconde extrémité opposée (60), la première extrémité (58) étant accouplée de façon mobile à la seconde extrémité (52) de la première portion (42) et la seconde extrémité (60) étant accouplée à une portion de l'organe à sangle (24) ;
dans lequel la deuxième portion (44) est mobile parmi une première position, dans laquelle les première et deuxième portions (42, 44) sont généralement alignées, et une seconde position, dans laquelle les première et deuxième portions (42, 44) sont disposées de façon généralement parallèle, et dans lequel le mécanisme de fixation (70) est disposé pour fixer sélectivement les première et deuxième portions (42, 44) dans la seconde position ;
dans lequel l'organe à sangle (24) est un organe à sangle continue (24), et dans lequel les première et deuxième portions (42, 44) sont sous forme de portions raidies ou renforcées de l'organe à sangle continue (24) ou placées par-dessus des portions de l'organe à sangle continue (24).

2. Ensemble cranio-cervical selon la revendication 1, dans lequel la première portion (42) du mécanisme d'ajustement (40, 40') comprend un nombre d'éléments rigides ou semi-rigides accouplés à, ou disposés dans ou à travers, une première portion de l'organe à sangle (24) ; dans lequel la deuxième portion (44) du mécanisme d'ajustement (40, 40') comprend un nombre d'éléments rigides ou semi-rigides accouplés à, ou disposés dans ou à travers, une deuxième portion de l'organe à sangle (24), et dans lequel la première extrémité (58) de la deuxième portion (44) du mécanisme d'ajustement (40, 40') est accouplée de façon mobile à la seconde extrémité (50) de la première portion (42) du mécanisme d'ajustement (40, 40') par une troisième portion de l'organe à sangle (24) disposée entre les première et deuxième portions (42, 44) de l'organe à sangle (24).

3. Ensemble cranio-cervical selon la revendication 1, dans lequel le mécanisme de fixation (70) comprend un nombre de composants coopérants (71) disposés d'une manière qui fixe sélectivement les première et deuxième portions (42, 44) du mécanisme d'ajustement (40, 40') dans la seconde position.

4. Ensemble cranio-cervical selon la revendication 3, dans lequel le nombre de composants coopérants (71) incluent des aimants et/ou des mécanismes à encliquetage.

5. Ensemble cranio-cervical selon la revendication 1, dans lequel le mécanisme de fixation (70) comprend un organe à sangle (72) accouplé au niveau d'une, ou sur une, parmi la première extrémité (50) de la première portion (42) et la seconde extrémité (60) de la deuxième portion (44) et sélectivement accouplé au niveau de l'autre, ou sur l'autre, parmi la première extrémité (50) de la première portion (42) et la seconde extrémité (60) de la deuxième portion (44).

6. Ensemble cranio-cervical selon la revendication 1, dans lequel le mécanisme d'ajustement (40') comprend une troisième portion (80) possédant une première extrémité (82) et une seconde extrémité opposée (84), la première extrémité (82) étant accouplée de façon mobile à la seconde extrémité (60) de la deuxième portion (44) du mécanisme d'ajustement (40').

7. Dispositif d'interface pour patient (10), comprenant :
(a) un ensemble d'étanchéité pour patient adapté pour faire communiquer un écoulement de gaz respiratoire avec des voies respiratoires d'un patient ; et
(b) un ensemble cranio-cervical (13) selon l'une quelconque des revendications 1 à 6 accouplé à l'ensemble d'étanchéité pour patient.

8. Dispositif d'interface pour patient selon la revendication 7, dans laquelle l'ensemble d'étanchéité pour patient comprend un masque (12) possédant une coque rigide (16) et un élément d'étanchéité (14) accouplé à la coque rigide (16), et dans laquelle la première extrémité (50) de la première portion (42) est accouplée à la coque rigide (16).
